# EUROPÄISCHE PATENTANMELDUNG

(11) **EP 2 306 183 A1**
(43) Veröffentlichungstag der Anmeldung: **06.04.2011**
(21) Anmeldenummer: 10450144.0
(22) Anmeldetag: 15.09.2010
(51) Int. Cl.: G01N 27/447, G01N 30/64

(54) **Probenanalysevorrichtung**

(30) Priorität: 16.09.2009 AT 14682009
(71) Anmelder: Integrated Microsystems Austria GmbH, 2700 Wiener Neustadt (AT); Technische Universität Wien, 1040 Wien (AT); Österreichische Akademie der Wissenschaften, 1010 Wien (AT)
(72) Erfinder: Fercher, Georg Dr., 1050 Wien (AT); Vellekoop, Michael J. Dr., 2371 Hinterbrühl (AT); Kohl, Franz Dr., 2345 Brunn am Gebirge (AT); Keplinger, Franz Dr., 1210 Wien (AT)
(74) Vertreter: Ellmeyer, Wolfgang

(57) **Zusammenfassung**

Probenanalysevorrichtung mit einem Separationskanal (1) zur Trennung von Probenkomponenten in Längsrichtung des Separationskanals (1) und mit im Bereich des Separationskanals (1) angeordneten Anregeelektroden (10, 12) zur Einkopplung eines Messsignals sowie Messelektroden (11, 13) zum Abgreifen eines Ausgangssignals, wobei die Anrege- und Messelektroden (10, 11, 12, 13) paarweise angeordnet sind, wobei zumindest zwei Paare von Anrege- und Messelektroden (10, 11, 12, 13) vorgesehen sind, und dass die zumindest zwei Paare von Anrege- und Messelektroden (10, 11, 12, 13) entlang der Länge des Separationskanals (1) voneinander beabstandet sind, wobei der Abstand (L1) zwischen den Paaren größer als der jeweilige Abstand (L2) zwischen den Anrege- und Messelektroden (10, 11, 12, 13) der Elektroden-Paare ist.

## Beschreibung

Die Erfindung betrifft eine Probenanalysevorrichtung mit einem Separationskanal zur Trennung von Probenkomponenten in Längsrichtung des Separationskanals und mit im Bereich des Separationskanals angeordneten Anregeelektroden zur Einkopplung eines Messsignals und Messelektroden zum Abgreifen eines Ausgangssignals.

Wichtige Methoden der analytischen Chemie, wie z.B. die Kapillarelektrophorese oder Chromatographie beruhen auf dem Prinzip der Trennung von Probenkomponenten durch Bewegen derselben in einem Separationskanal, der durch flüssigkeitsführende Kapillaren, Mikrokanäle oder Säulen gebildet sein kann.

Aufgrund der unterschiedlichen Ladungen und Massen der Probenkomponenten stellen sich dabei unterschiedliche Wanderungsgeschwindigkeiten durch den Separationskanal ein, die eine Trennung bewirken. Die Messung der elektrischen Leitfähigkeit stellt eine Methode dar, um leitfähige Probenkomponenten, wie z.B. Ionen zu bestimmen. Üblicherweise ist der Messbereich im Bereich des Endes des Separationskanals angeordnet, an dem ein Messsignal eingekoppelt und abgegriffen wird. Es werden Änderungen im Ausgangsstrom aufgrund der durch die in den separierten Zonen auftretenden Leitfähigkeitsschwankungen gemessen. Die dabei verwendeten Elektroden können entlang des Separationskanals oder quer zu diesem angeordnet sein und verschiedenste Ausführungsformen aufweisen.

Das alternierende Messsignal wird an einer Anregeelektrode in einen kurzen Bereich des Separationskanals eingekoppelt und an einer unmittelbar daneben angeordneten Messelektrode abgegriffen. Bei herkömmlichen Elektrodenanordnungen kommt es durch parasitäre Streukapazitäten und die Hintergrundleitfähigkeit der verwendeten Pufferlösung zu einem sehr hohen Grundpegel des Ausgangssignals. Temperaturdriften und Rauschen im Grundpegel können zu Verfälschungen der gemessenen Signalpeaks und zu verschlechterten Messempfindlichkeiten führen. Weiters muss der Eingangsdynamikbereich der Auswerteelektronik auf einen dem Grundpegel entsprechenden hohen Wert eingestellt werden, was sich negativ auf das Signal/Rauschverhältnis und somit wiederum auf die Messempfindlichkeit auswirkt.

Aufgabe der Erfindung ist es daher, eine Probenanalysevorrichtung der eingangs genannten Art anzugeben, mit der eine Erhöhung der Messempfindlichkeit für das Ausgangssignal sowie eine Verbesserung der räumlichen Auflösung erzielt werden können.

Weitere Aufgabe der Erfindung ist es, eine Bestimmung der während der Messung auftretenden Diffusions- und Reibungseffekte der separierten Probenkomponenten zu ermöglichen.

Erfindungsgemäß wird dies dadurch erreicht, dass die Anrege- und Messelektroden paarweise angeordnet sind, wobei zumindest zwei Paare von Anrege- und Messelektroden vorgesehen sind, und dass diese zumindest zwei Paare von Anregeund Messelektroden entlang der Länge des Separationskanals voneinander beabstandet sind, wobei der Abstand zwischen den Paaren größer als der jeweilige Abstand zwischen den Anrege- und Messelektroden der Elektroden-Paare ist.

Über die zwei oder mehreren Anrege- und Messelektroden, die an unterschiedlichen Stellen des Separationskanals angeordnet sind, kann das oder die Messsignale eingekoppelt und an den zugehörigen, unmittelbar benachbarten Messelektroden können die Ausgangssignale abgegriffen werden. Es entstehen somit unabhängig voneinander erzeugte zwei oder mehrere Ausgangssignale, die für eine differentielle Messung herangezogen werden können. Durch geeignete Anordnung der Elektrodenpaare kann somit der relativ hohe Grundpegel des Ausgangssignals beseitigt und auf diese Weise die Messempfindlichkeit negativ beeinflussende Störgrößen minimiert werden. Da die Einkopplung des Messsignals an unterschiedlichen Stellen des Separationskanals erfolgt, wird die differentielle Messung auf separat erzeugte Ausgangssignale gestützt, die eine höhere räumliche Auflösung für die sich an den Elektroden vorbeibewegenden Probenkomponenten ermöglichen, woraus sich eine geringe Breite und größere Höhe der Signalpeaks ergibt.

Zusätzlich können aus dem Vergleich der Ausgangssignale der Messelektroden Aussagen über Diffusions- und Reibungseigenschaften der sich im Kanal bewegenden Probenkomponenten getroffen werden, die zur Signalverarbeitung verwendet werden können.

Gemäß der Erfindung sind zumindest zwei Elektrodenpaare zur Bestimmung der Probenkomponenten vorgesehen, es können aber auch mehrere solcher Paare entlang des Kanalverlaufes angeordnet sein. Sowohl die Geometrie des Separationskanals als auch die der Anrege- und Messelektroden kann im Rahmen der Erfindung variieren. Auch hinsichtlich der Anwendung der erfindungsgemäßen Probenanalysevorrichtung im Bereich der analytischen Chemie bestehen keine Einschränkungen, vielmehr kann diese in allen Bereichen eingesetzt werden, wo eine Leitfähigkeitsmessung in einem Separationskanal zur Bestimmung von Probenkomponenten möglich ist. Auch hinsichtlich der Materialwahl sowohl des Separationskanals als auch der Anrege- und Messelektroden und des Aufbaus der Signalauswerteschaltung gibt es keine Einschränkungen.

Gemäß einem Ausführungsbeispiel der Erfindung kann ein erstes und ein zweites Paar von Anrege- und Messelektroden vorgesehen sein, wobei das erste Paar von Anrege- und Messelektroden - in Bewegungsrichtung der Probenkomponenten gesehen - im Anfangsbereich und das zweite Paar von Anrege- und Messelektroden im Endbereich des Separationskanals angeordnet sind. Über die im Anfangs- und Endbereich vorgesehenen Elektrodenpaare werden zwei Ausgangssignale erzeugt, aus denen ein vom Grundpegel befreites Elektropherogramm gewonnen werden kann.

Um eine differentielle Auswertung der an den Elektrodenpaaren erzeugten Ausgangssignale zu erreichen, können die Messelektroden der zumindest zwei Paare von Anrege- und Messelektroden mit den Eingängen einer Subtrahierschaltung verbunden sein, deren Ausgangssignal zu den von einander subtrahierten Ausgangssignalen der Messelektroden proportional ist. Zur besseren Signalverarbeitung kann zwischen den Messelektroden und den Eingängen der Subtrahierschaltung jeweils ein Vorverstärker geschaltet sein.

Gemäß einer weiteren Ausführungsform der Erfindung können auch zumindest zwei Paare von Anrege- und Messelektroden vorgesehen sein, die jeweils ein Glied einer Brückenschaltung bilden, und die beiden anderen Brückenglieder können durch zwei Impedanzen gebildet sein, wobei der Verbindungspunkt der miteinander verbundenen Messelektroden und der Verbindungspunkt der an einem Ende miteinander verbundenen Impedanzen mit den Eingängen einer Subtrahierschaltung verbunden sind, und wobei die mit dem jeweils anderen Ende der beiden Impedanzen verbundenen Anregeelektroden jeweils mit einer Messspannungsquelle verbunden sind, wobei diese Messspannungsquellen Messspannungen erzeugen, die zueinander phasenverschoben sind.

Bei Anwendung einer kontaktlosen Leitfähigkeitsmessung bei der Anwendung der erfindungsgemäßen Probenanalysevorrichtung können die Anrege- und die Messelektroden zur kontaktlosen Leitfähigkeitsmessung gegenüber der Innenwand des Separationskanals nach außen versetzt sein. Dadurch ist eine Isolation der Anrege- und Messelektroden gegenüber der im Separationskanal befindlichen Pufferlösung sichergestellt, wodurch chemische Reaktionen der Elektroden mit den Pufferlösung oder der zu analysierenden Probe vermieden werden.

Insbesondere für die Realisierung der erfindungsgemäßen Probenanalysevorrichtung als Mikrofluidik-System ist eine schichtenförmige Anordnung des Separationskanals und der Anrege- und Messelektroden von Vorteil.

Somit kann es daher vorteilhaft sein, wenn die Anrege- und die Messelektroden der zumindest zwei Elektroden-Paare in einer parallel zum Separationskanal verlaufenden Ebene angeordnet sind. Dadurch ergibt sich ein relativ einfacher Aufbau.

Eine mögliche Ausführungsform der Erfindung kann darin bestehen, dass die Anrege- und die Messelektroden der zumindest zwei Elektroden-Paare oberhalb des Separationskanals ausgebildet sind. Dies ermöglicht einen schichtweisen Aufbau der Probenanalysevorrichtung, bei dem nach der Ausbildung des Separationskanals in einer Substratschicht eine darauffolgende weitere Substratschicht aufgetragen wird, auf welcher die Anrege- und Messelektroden zusammen mit den erforderlichen Anschlussleitungen an den geeigneten Stellen höhenversetzt zum Separationskanal vorgesehen sind.

Weiters kann in diesem Zusammenhang vorgesehen sein, dass die Anrege- und Messelektroden der zumindest zwei Elektroden-Paare in Längsrichtung des Separationskanals fluchtend angeordnet sind, sodass sich alle Elektroden genau höhenversetzt zum Verlauf des Separationskanal erstrecken.

Eine andere Ausführungsform der Erfindung kann darin bestehen, dass die Anregeund Messelektroden der zumindest zwei Elektroden-Paare an einander gegenüberliegenden Seiten des Separationskanals angeordnet sind, sodass die Anrege- und Messelektroden jedes Elektroden-Paares an gegenüberliegenden Stellen angebracht sind, um die Einkopplung eines elektrischen Feldes zu ermöglichen, das sich quer zur Längsrichtung durch den Separationskanal hindurch erstreckt.

Die Homogenität des zur Leitfähigkeitsmessung erzeugten elektrischen Feldes im Separationskanal hängt stark von der Geometrie der Anrege- und Messelektroden ab. Als nachteilig bei der gegenüberliegenden Anordnung der Anrege- und Messelektroden hat es sich herausgestellt, dass es zu Ausbauchungen des elektrischen Feldes kommt, welche den Messbereich in unerwünschter Weise vergrößern. Es entstehen Verbreiterungen der erzeugten Ausgangs-Signalpeaks und Stromableitungen, die zu Verfälschungen des Ausgangssignals führen.

Um diesen Effekt zu vermeiden, können gemäß einer Weiterbildung der Erfindung benachbart zu den Anrege- und Messelektroden und an einander gegenüberliegenden Seiten des Separationskanals Guard-Elektroden angeordnet sein, welche mit einer Referenzspannungsquelle verbindbar sind. Die Guard-Elektroden stellen ein homogenes elektrisches Feld im Messbereich sicher, indem sie die Ausbuchtungen des Feldes reduzieren. Seitliche Stromableitungen werden auf diese Weise ebenso vermieden. Lokale Leitfähigkeitsänderungen infolge der separierten, leitfähigen Analyten können so mit höherer räumlicher Auflösung gemessen werden. Mit anderen Worten können räumliche Abstände zwischen den separierten Probenkomponenten einer Probe genauer bestimmt werden.

Die Guard-Elektroden werden bevorzugt auf dem selben Potenzial wie die benachbarten Anrege- und Messelektroden gehalten und sie umschließen die Anrege- und Messelektroden oder sind an gegenüberliegenden Seiten dieser angeordnet.

Für einen schichtweisen Aufbau der erfindungsgemäßen Probenanalysevorrichtung ist es daher vorteilhaft, wenn gemäß einer Ausführungsform der Erfindung ein Teil der Guard-Elektroden mit den Anregeelektroden und ein weiterer Teil der Guard-Elektroden mit den Messelektroden in jeweils einer Ebene angeordnet sind.

Aus produktionstechnischen Gründen hat es sich weiters als vorteilhaft erwiesen, wenn die Guard-Elektroden sowie die Anrege- und Messelektroden entlang der Längsrichtung des Separationskanals angeordnet sind. Es kann im Rahmen der Erfindung jede andere realisierbare Geometrie von Guard-, Anrege- und Messelektroden gewählt werden.

Für den schichtweisen Aufbau, z.B. in einer Mikrofluidikvorrichtung, können die Anrege- und Messelektroden sowie die Guard-Elektroden planar ausgebildet sein.

Für die Bereitstellung einer kontaktlosen Leitfähigkeitsmessung können weiters die Anrege- und Messelektroden und/oder die Guard-Elektroden in der Wand des Separationskanals eingebettet sein.

Grundsätzlich ist die erfindungsgemäße Probenanalysevorrichtung für verschiedene Trennverfahren geeignet, für welche eine oder mehrere Pufferlösungen erforderlich sind.

Es kann daher ein Probeneinlass und/oder ein Injektionskanal zum Einbringen der zu analysierenden Probe vorgesehen sein, der mit dem Separationskanal verbunden ist. Sobald der Vorgang des Einbringens der Probe beendet ist, erfolgt das Anlegen einer Gleichspannung, z.B. an den Enden des Separationskanals um die Wanderbewegung der Probenkomponenten je nach Ladung und Masse in Gang zu setzen.

Bei einer Ausführungsform der erfindungsgemäßen Probenanalysevorrichtung kann der Probeneinlass im Anfangsbereich des Separationskanals angeordnet sein, von wo aus die Probenkomponenten aufgrund der angelegten Gleichspannung in Längsrichtung des Separationskanals bewegt werden, um bei Vorbeiwandern an den paarweise angeordneten Anrege- und Messelektroden eine messbare Leitfähigkeitsschwankung zu erzeugen.

Es kann gemäß einer anderen Ausführungsform der Erfindung der Probeneinlass ungefähr in der Längsmitte des Separationskanals angeordnet und zwischen dem Probeneinlass und den beiden Enden des Separationkanals jeweils zumindest ein Paar von Anrege- und Messelektroden angeordnet sein. Eine solche Anordnung ermöglicht eine Injektion der Probe im mittleren Bereich des Separationskanals, von wo aus die Probenkomponenten bei geeignet angelegter Gleichspannung in entgegengesetzte Richtungen zu dem jeweiligen Ende des Separationskanals bewegt werden. Somit kann eine Bewegung in entgegensetzten Richtungen erfolgen und an den zu beiden Seiten des Probeneinlasses vorgesehenen Anrege- und Messelektroden kann jeweils zur gleichen Zeit ein Ausgangssignal erzeugt werden, welches zur differentiellen Signalverwertung geeignet ist. Weiters ermöglicht ein ungefähr in Längsmitte des Separationskanals angeordneter Probeneinlass die gleichzeitige Separation von positiv und negativ geladenen Analyten, da diese nun ihrer Ladung entsprechend zum jeweiligen Ende des Separationskanals wandern und währenddessen voneinander separiert werden.

Die Erfindung betrifft weiters die Verwendung einer Probenanalysevorrichtung in einem Elektrophoreseverfahren oder in einem Isotachophoreseverfahren. Weitere ähnliche Anwendungen liegen ebenfalls im Schutzbereich der Erfindung.

Die Erfindung betrifft weiters ein Verfahren zur Analyse von Proben durch Auftrennung von Probenkomponenten in einem Separationskanal, wobei in den Separationskanal zumindest ein Messsignal eingekoppelt wird und zumindest ein Ausgangssignal abgegriffen wird.

Die eingangs genannten Aufgaben werden bei einem vorgenannten erfindungsgemäßen Verfahren erfindungsgemäß dadurch erreicht, dass zumindest ein Messsignal an zumindest zwei in Längsrichtung des Separationskanals voneinander beabstandeten Stellen in den Separationskanal eingekoppelt und dass zumindest zwei Ausgangssignale abgegriffen werden, die voneinander subtrahiert werden.

Nachfolgend wird die Erfindung anhand der in den Zeichnungen dargestellten Ausführungsbeispiele eingehend erläutert. Es zeigt dabei
Fig.1 eine Explosionsdarstellung einer Ausführungsform der erfindungsgemäßen Probenanalysevorrichtung;
Fig.2 eine teilweise schematische Draufsicht auf die Probenanalysevorrichtung gemäß Fig.1 mit einem Schaltbild einer zugehörigen Signalverarbeitungsschaltung;
Fig.3 einen Schnitt durch die Probenanalysevorrichtung gemäß Fig.1 durch den Separationskanal und in dessen Längsrichtung;
Fig.4 eine teilweise schematische Draufsicht auf die Probenanalysevorrichtung gemäß Fig.1;
Fig.5 einen Schnitt durch die Probenanalysevorrichtung gemäß Fig.1 durch den Separationskanal und in dessen Längsrichtung;
Fig.6 eine teilweise schematische Draufsicht auf eine weitere Ausführungsform der erfindungsgemäßen Vorrichtung;
Fig.7 einen Schnitt durch die Probenanalysevorrichtung gemäß Fig.6
Fig.8 einen Schnitt durch eine weitere Ausführungsform der erfindungsgemäßen Probenanalysevorrichtung mit einem Schaltbild einer Signalverarbeitungsvorrichtung;
Fig.9 einen Schnitt durch eine weitere Ausführungsform der erfindungsgemäßen Probenanalysevorrichtung mit einem Schaltbild einer Signalverarbeitungsvorrichtung;
Fig.10 einen Schnitt durch eine weitere Ausführungsform der erfindungsgemäßen Probenanalysevorrichtung mit einem Schaltbild einer Signalverarbeitungsvorrichtung;
Fig.11 einen Schnitt durch eine weitere Ausführungsform der erfindungsgemäßen Probenanalysevorrichtung mit einem Schaltbild einer Signalverarbeitungsvorrichtung;
Fig.12 einen Schnitt durch eine weitere Ausführungsform der erfindungsgemäßen Probenanalysevorrichtung mit einem Schaltbild einer Signalverarbeitungsvorrichtung;
Fig.13 einen Schnitt durch eine weitere Ausführungsform der erfindungsgemäßen Probenanalysevorrichtung mit einem Schaltbild einer Signalverarbeitungsvorrichtung;
Fig.14 einen Schnitt durch eine weitere Ausführungsform der erfindungsgemäßen Probenanalysevorrichtung mit einem Schaltbild einer Signalverarbeitungsvorrichtung;
Fig.15 ein mit einer Probenanalysevorrichtung gemäß Stand der Technik aufgenommenes Elektropherogramm;
Fig.16 ein mit einer erfindungsgemäßen Probenanalysevorrichtung aufgenommenes Elektropherogramm;

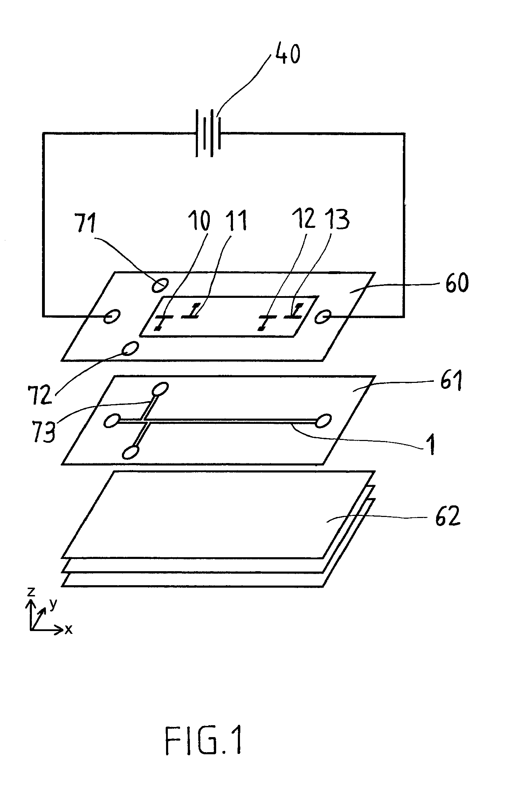

Fig. 1 bis 5 zeigen eine Probenanalysevorrichtung zur kapillaren Zonen-Elektrophorese, die als integrierter Analysechip-Bauteil ausgebildet ist, die aus mehreren Substratschichten 60, 61 und 62 (Fig.1) zu einem Substrat 90 zusammengesetzt ist.

In der Substratschicht 61 ist ein gerader Separationskanal 1, der auch gewunden, meanderförmig etc. ausgebildet sein kann, ausgenommen. Typische Kanaldurchmesser sind z.B. 5 bis 150 Mikrometer. Der Kanalquerschnitt kann beliebig geformt, z.B. rund, quadratisch, rechteckig, grubenförmig etc. sein.

Beim Messvorgang wird der Separationskanal 1 zunächst mit einer geeigneten Pufferlösung befüllt. Dann wird die zu analysierende Probe an einem Ende über einen Probeneinlass 71 und einen Injektionskanal 73, z.B. in Form einer Double-T-oder Cross-T-Injektion, in den Separationskanal 1 eingebracht.

Nach erfolgter Probeninjektion wird eine geeignete Separationsspannung, vorzugsweise eine Gleichspannung, über eine Spannungsquelle 40 an den Enden des Separationskanals 1 angelegt und somit der elektrophoretische Separationsvorgang gestartet. Die Richtung der daraus resultierenden, elektrischen Feldlinien wird dem Anwendungsfall entsprechend gewählt, z.B. zur Trennung von Probenkomponenten wie Anionen und Kationen.

Die in der Probe enthaltenen Ionen wandern nun in dem von der Spannungsquelle 40 erzeugten elektrischen Feld von dem einen Ende des Separationskanals 1 zum anderen und werden dabei in Längsrichtung des Separationskanals 1 separiert.

Weiters sind im Bereich des Separationskanals 1 Anregeelektroden 10, 12 zum Einkoppeln eines Messsignals und Messelektroden 11, 13 zum Abgreifen eines Ausgangssignals angeordnet.

Erfindungsgemäß sind die Anrege- und Messelektroden 10, 11, 12, 13 paarweise angeordnet, wobei im gezeigten Ausführungsbeispiel ein erstes und ein zweites Paar von Anrege- und Messelektroden 10, 11, 12, 13 vorgesehen sind, wobei das erste Paar von Anrege- und Messelektroden 10, 11 - in Bewegungsrichtung der Probenkomponenten gesehen - im Anfangsbereich und das zweite Paar von Anregeund Messelektroden 12, 13 im Endbereich des Separationskanals 1 angeordnet sind.

Jedes Elektroden-Paar setzt sich aus einer Anregeelektrode 10 bzw. 12 und einer Messelektrode 11 bzw.13 zusammen und bildet jeweils einen Detektor.

Die zwei Paare von Anrege- und Messelektroden 10, 11, 12, 13 sind planar ausgebildet und entlang der Länge des Separationskanals 1 voneinander beabstandet, wobei der Abstand L1 zwischen den Paaren größer als der jeweilige Abstand L2 zwischen den Anrege- und Messelektroden 10, 11, 12, 13 der Paare ist.

Über eine Wechselspannungsquelle 29, die mit den Anregeelektroden 10, 12 beider Elektrodenpaare verbunden ist, wird ein alternierendes Messsignal an den Anregeelektroden 10, 12 jeweils in einen kurzen Bereich des Separationskanals 1 eingekoppelt und an den zugehörigen Messelektroden 11, 13 der Elektrodenpaare abgegriffen.

Somit befinden sich die Elektroden-Paare an unterschiedlichen Stellen des Separationskanals 1 und das Messsignal wird an zwei Stellen in den Separationskanal 1 eingekoppelt und jeweils an der unmittelbar benachbarten Messelektrode des Elektroden-Paars abgegriffen. Es entstehen somit zwei Ausgangssignale.

Die Messelektroden 11, 13 der beiden Paare sind über einen Vorverstärker 21, 22 mit den Eingängen einer Subtrahierschaltung 20 verbunden, deren Ausgangssignal zu den von einander subtrahierten Ausgangssignalen der Messelektroden 11, 13 proportional ist.

Das Ausgangssignal der Subtrahierschaltung 20 wird vorzugsweise in einem Lock-in-Verstärker 25 verstärkt und vorzugsweise an einem Rechner 26 zur Anzeige gebracht.

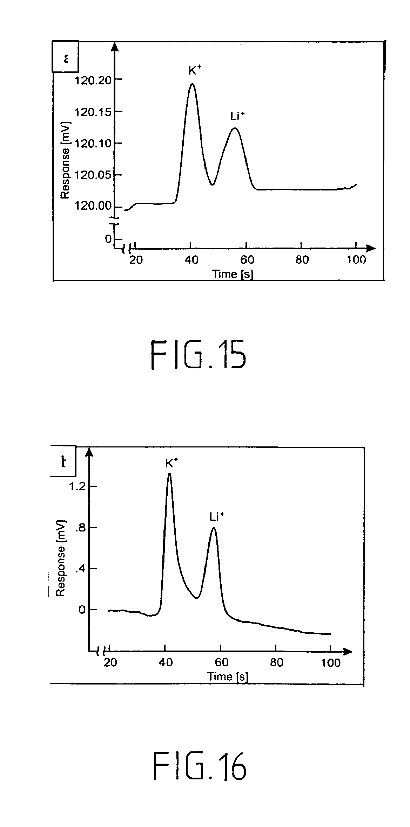

Bei einer Anordnung der Anrege- und Messelektroden gemäß Stand der Technik kommt es infolge parasitärer Streukapazitäten und der Hintergrundleitfähigkeit der Pufferlösung zu einem sehr hohen Grundpegel im Ausgangssignal, wie aus dem in Fig.15 gezeigten Ausgangssignal einer erfolgreichen Separation der Ionen Kalium und Lithium zu ersehen ist. Diese beiden Ionen konnten innerhalb eines Zeitraumes von ca. 60 Sekunden separiert werden. Der Grundpegel (Baseline) liegt hier bei ca. 120 mV, während der interessierende Ausschlag des Signals maximal 200 µV beträgt, also nur ca. 0,2% des gesamten Signals. Temperaturdriften im Grundpegel, aber auch Rauschen können dadurch zu Verfälschungen der gemessenen Signalpeaks oder verschlechterten Messempfindlichkeiten führen. Weiters muss der Eingangsdynamikbereich der Auswerteelektronik auf einen dem Grundpegel entsprechenden Wert gesetzt werden, was sich negativ auf das Signal/Rauschverhältnis und somit wiederum auf die Messempfindlichkeit auswirkt.

Fig.16 zeigt den Verlauf des Messsignals für die erfindungsgemäße Probenanalysevorrichtung mit differenzieller Messung, mit der der Grundpegel kompensiert werden kann. Es ergeben sich wesentlich schärfere Signalmaxima.

Weiters kann die erfindungsgemäße Probenanalysevorrichtung zur Abschätzung von auf die separierten Probenkomponenten einwirkenden Diffusionseffekte herangezogen werden. Die zu analysierende Probe wandert zuerst an dem sich im Anfangsbereich des Separationskanals 1 befindlichen Elektrodenpaar 10, 11 vorbei. Es ergibt sich ein differenzielles Ausgangssignal bestimmter Breite, welches mit der Breite der Ionenverteilung im Separationskanal 1 korrespondiert. Wandert nun diese Probe weiter und passiert schließlich das im Endbereich des Separationskanals 1 befindliche Elektroden-Paar 12, 13 ergibt sich wiederum ein differenzielles Ausgangssignal, das in der Regel breiter sein wird als das anfangs gemessene. So können die auf die Probe einwirkenden Diffusionseffekte (Reibungseffekte) bestimmt und eventuell kompensiert werden. Dies ist vor allem für intransparente Chipmaterialien nützlich, bei denen optische Verfahren nicht angewandt werden können.

Die in den Ausführungsbeispielen gemäß Fig.1 bis 7 gewählte Anordnung des Separationskanals und der Elektrodenpaare kann im Rahmen der Erfindung variieren. Es können beliebig weitere Elektrodenpaare vorgesehen sein.

Weiters kann die erfindungsgemäße Probenanalysevorrichtung für verschiedene Separationsverfahren angewandt werden, etwa Elektrophorese oder Chromatographie. Die Anordnung der Elektroden kann dabei so sein, dass sowohl kontaktlose als auch kontaktierende Messung der Leitfähigkeit möglich ist.

Bevorzugt ist die erfindungsgemäße Probenanalysevorrichtung als Mikrofluidikvorrichtung realisiert, sie kann aber auch in einem konventionell aufgebauten Probenanalysesystem mit Kapillaren vorgesehen sein.

Bei der Ausführungsform gemäß Fig.1 bis 5 sind die Anrege- und die Messelektroden 10, 11, 12, 13 zur kontaktlosen Leitfähigkeitsmessung gegenüber der Innenwand des Separationskanals 1 nach außen versetzt. Die Messsignale werden somit durch kapazitive Kopplung in den Separationskanal 1 eingespeist und Ausgangssignale abgegriffen.

Weiters sind die Anrege- und die Messelektroden 10, 11, 12, 13 der zwei Elektroden-Paare in einer parallel zum Separationskanal 1 verlaufenden Ebene oberhalb desselben angeordnet und können somit in und auf einer einzigen Substratschicht 60 (Fig.1) ausgebildet sein. Entsprechende Anschlusskontaktstellen können jeweils über leiterbahnartige Verbindungen vorgesehen sein.

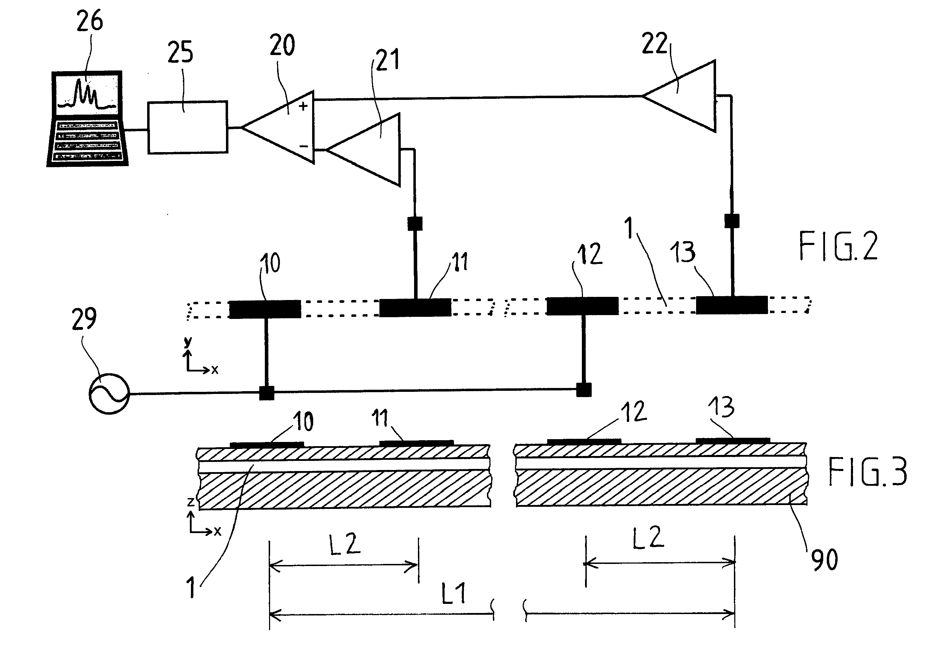

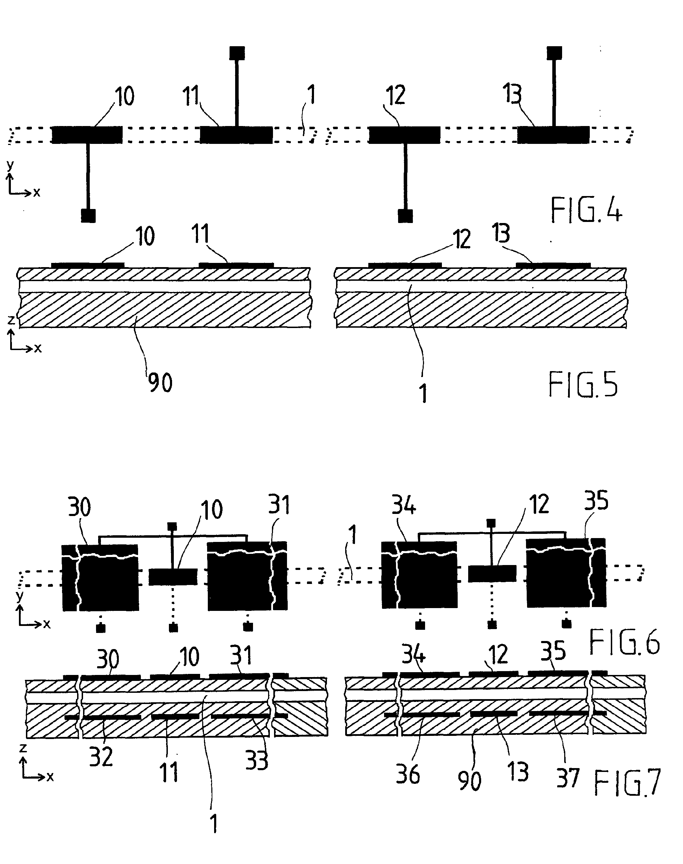

Wie aus Fig.2 und 4 ersichtlich sind die Anrege- und Messelektroden 10, 11, 12, 13 der Elektrodenpaare in Längsrichtung des Separationskanals 1 fluchtend angeordnet und weisen ungefähr die Breite des Separationskanals 1 auf, während sie sich in dessen Längsrichtung über mehr als die doppelte Breite des Separationskanals 1 erstrecken.

In Fig.6 und 7 sind die Anrege- und Messelektroden 10, 11, 12, 13 der zwei Elektroden-Paare an einander gegenüberliegenden Seiten des Separationskanals 1 in oberhalb und unterhalb des Separationskanals 1 verlaufenden Ebenen angeordnet. Das elektrische Feld der Anregeelektroden 10, 12 erstreckt sich daher quer zum Separationskanal 1 durch diesen hindurch zu den Messelektroden 11, 13. Um eine seitliche Ausbauchung des Feldes und Ableitströme niedrig zu halten, können an den Stirnseiten der Elektrodenpaare jeweils benachbart zu den Anregeund Messelektroden 10, 11, 12, 13 und an einander gegenüberliegenden Seiten des Separationskanals 1 Guard-Elektroden 30, 31, 32, 33 sowie 34, 35, 36, 37 angeordnet sein, welche mit einer Referenzspannungsquelle verbindbar sind.

Die quadratischen Guard-Elektroden 30, 31, 32, 33 sowie 34, 35, 36, 37 sind in der Ebene der Anrege- und Messelektroden 10, 11, 12, 13 angeordnet und haben eine Kantenlänge, die ungefähr der vierfachen Breite des Separationskanals 1 entspricht.

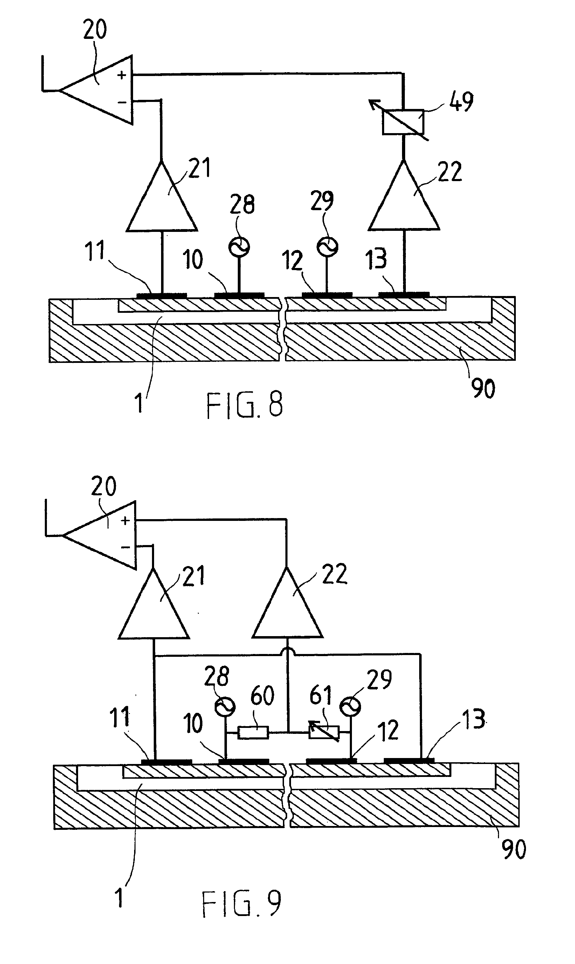

Fig.8 zeigt eine erfindungsgemäße Probenanalysevorrichtung mit differentieller Messanordnung, bei der die Anregeelektroden 10, 12 der zwei Elektrodenpaare durch zwei Spannungsquellen 28, 29 voneinander getrennt gespeist sind. Über eine Ausgleichsschaltung 49, die zwischen der Messelektrode 13 des zweiten Elektrodenpaars und dem Eingang der Subtrahierschaltung 20 geschaltet ist, können Pegeldifferenzen ausgeglichen werden, um eine bestmögliche Empfindlichkeit der Anordnung erzielen zu können.

Fig.9 zeigt eine differentielle Messanordnung mit Brückenbeschaltung, bei der zwei Paare von Anrege- und Messelektroden 10, 11 sowie 12, 13 vorgesehen sind, die jeweils ein Glied einer Brückenschaltung bilden. Die beiden anderen Brückenglieder sind durch zwei Impedanzen 60, 61 gebildet. Der Verbindungspunkt der miteinander verbundenen Messelektroden 11, 13 und der Verbindungspunkt der an einem Ende miteinander verbundenen Impedanzen 60, 61 sind über Vorverstärker 21, 22 mit den Eingängen der Subtrahierschaltung 20 verbunden. Die mit dem jeweils anderen Ende der beiden Impedanzen 60, 61 verbundenen Anregeelektroden 10, 12 sind jeweils mit einer Messsignalquelle 28, 29 verbunden sind, wobei diese Messsignalquellen 28, 29 Messspannungen erzeugen, die zueinander phasenverschoben sind.

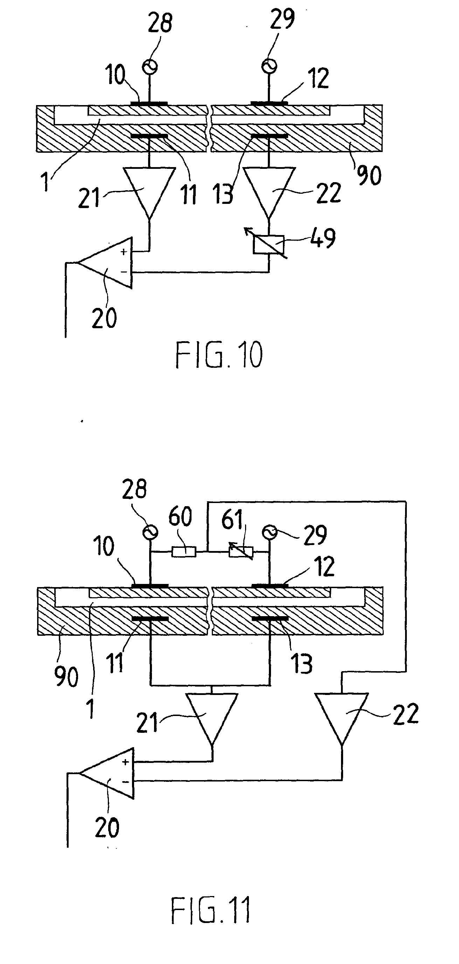

Fig. 10 zeigt eine Ausführungsbeispiel der Erfindung mit paarweiser Anordnung der Anrege- und Messelektroden 10, 11, 12, 13, wobei die Anregeelektroden und die Messelektroden jedes Elektrodenpaars einander gegenüberliegend angeordnet sind. Die Signalauswerteschaltung entspricht jener des in Fig. 8 gezeigten Ausführungsbeispiels.

Im Ausführungsbeispiel gemäß Fig.11 wird die in Fig.9 gezeigte Brückenbeschaltung auf eine gegenüberliegende Anordnung der Anrege- und Messelektroden 10, 11, 12, 13 angewandt.

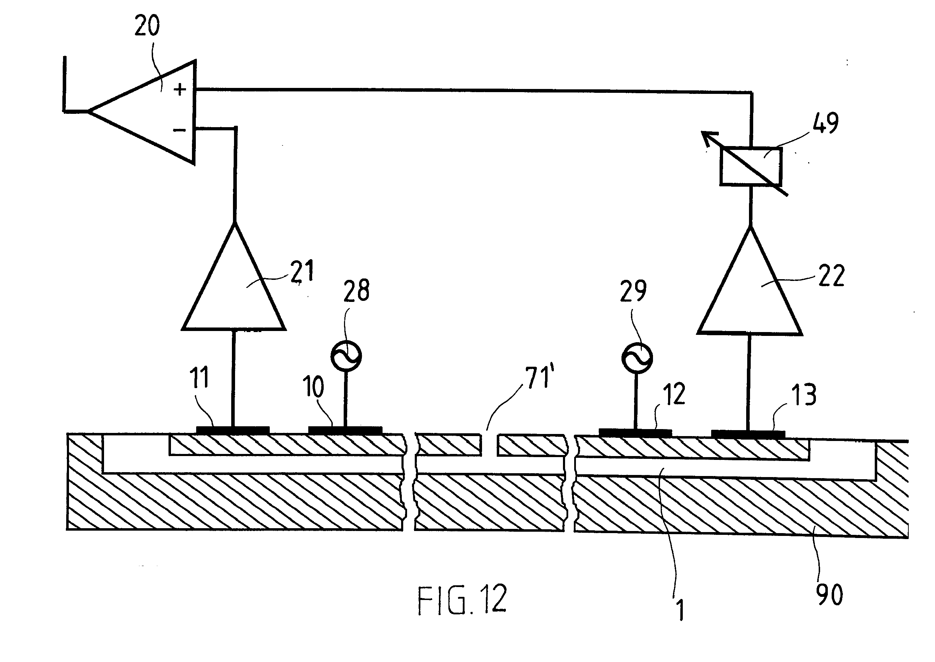

Beim Ausführungsbeispiel gemäß Fig. 12 ist der Probeneinlass 71' ungefähr in der Längsmitte des Separationskanals 1 angeordnet, wobei zwischen dem Probeneinlass 71' und den beiden Enden des Separationkanals 1 jeweils zumindest ein Paar von Anrege- und Messelektroden 10, 11, 12, 13 angeordnet ist. Wird über den mittigen Probeneinlass 71' eine Probe eingebracht, so bewegt sich diese aufgrund einer geeignet angelegten Gleichspannung in entgegensetzten Richtungen zu den beiden Enden des Separationskanals 1. In beiden Bewegungsrichtungen werden bei Vorbeibewegen der separierten Probenkomponenten entsprechende Leitfähigkeitspeaks über die Messelektroden 11, 13 aufgenommen und als Differenzsignale in der Subtrahierschaltung voneinander abgezogen.

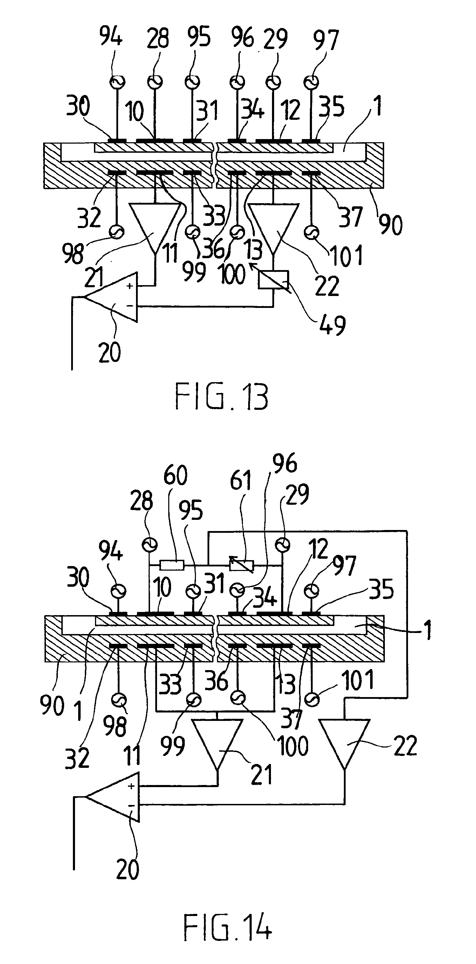

Fig.13 zeigt das Ausführungsbeispiel gemäß Fig.10 mit zusätzlichen Guard-Elektroden 30, 31, 32, 33, 34, 35, 36, 37, wie sie in Fig.6, 7 gezeigt sind. Die Guard-Elektroden 30, 31, 32, 33, 34, 35, 36, 37, weiche für eine homogene Feldausbreitung zwischen den Anrege- und Messelektroden 10, 11, 12, 13 sorgen, sind mit separaten Spannungsquellen 94, 95, 98, 99, 96, 97, 100, 101 verbunden, auf welche aber durch geeignete Zusammenschaltung auch verzichtet werden kann.

Fig.14 zeigt das Ausführungsbeispiel gemäß Fig.11 mit zusätzlichen Guard-Elektroden 30, 31, 32, 33, 34, 35, 36, 37, wie sie in Fig.6, 7 gezeigt sind. Die Guard-Elektroden 30, 31, 32, 33, 34, 35, 36, 37, welche für eine homogene Feldausbreitung zwischen den Anrege- und Messelektroden 10, 11, 12, 13 sorgen, sind mit separaten Spannungsquellen 94, 95, 98, 99, 96, 97, 100, 101 verbunden, auf welche aber durch geeignete Zusammenschaltung zumindest teilweise verzichtet werden kann.

## Patentansprüche

1. Probenanalysevorrichtung mit einem Separationskanal (1) zur Trennung von Probenkomponenten in Längsrichtung des Separationskanals (1) und mit im Bereich des Separationskanals (1) angeordneten Anregeelektroden (10, 12) zur Einkopplung eines Messsignals sowie Messelektroden (11, 13) zum Abgreifen eines Ausgangssignals, **dadurch gekennzeichnet, dass** die Anrege- und Messelektroden (10, 11, 12, 13) paarweise angeordnet sind, wobei zumindest zwei Paare von Anrege- und Messelektroden (10, 11, 12, 13) vorgesehen sind, und dass die zumindest zwei Paare von Anrege- und Messelektroden (10, 11, 12, 13) entlang der Länge des Separationskanals (1) voneinander beabstandet sind, wobei der Abstand (L1) zwischen den Paaren größer als der jeweilige Abstand (L2) zwischen den Anrege- und Messelektroden (10, 11, 12, 13) der Elektroden-Paare ist.

2. Probenanalysevorrichtung nach Anspruch 1, **dadurch gekennzeichnet, dass** ein erstes und ein zweites Paar von Anrege- und Messelektroden (10, 11, 12, 13) vorgesehen sind, und dass das erste Paar von Anrege- und Messelektroden (10, 11) - in Bewegungsrichtung der Probenkomponenten gesehen - im Anfangsbereich und das zweite Paar von Anrege- und Messelektroden (12, 13) im Endbereich des Separationskanals (1) angeordnet sind.

3. Probenanalysevorrichtung nach Anspruch 1 oder 2, **dadurch gekennzeichnet, dass** die Messelektroden (11, 13) der zumindest zwei Paare von Anrege- und Messelektroden (10, 11, 12, 13) mit den Eingängen einer Subtrahierschaltung (20) verbunden sind, deren Ausgangssignal zu den von einander subtrahierten Ausgangssignalen der Messelektroden (11, 13) proportional ist.

4. Probenanalysevorrichtung nach Anspruch 3, **dadurch gekennzeichnet, dass** zwischen den Messelektroden (11, 13) und den Eingängen der Subtrahierschaltung (20) jeweils ein Vorverstärker (21, 22) geschaltet ist.

5. Probenanalysevorrichtung nach einem der vorhergehenden Ansprüche, **dadurch gekennzeichnet, dass** zwei Paare von Anrege- und Messelektroden (10, 11, 12, 13) vorgesehen sind, die jeweils ein Glied einer Brückenschaltung bilden, und dass die beiden anderen Brückenglieder durch zwei Impedanzen (60, 61) gebildet sind, wobei der Verbindungspunkt der miteinander verbundenen Messelektroden (11, 13) und der Verbindungspunkt der an einem Ende miteinander verbundenen Impedanzen (60, 61) mit den Eingängen einer Subtrahierschaltung (20) verbunden sind, und wobei die mit dem jeweils anderen Ende der beiden Impedanzen verbundenen Anregeelektroden (10, 12) jeweils mit einer Messspannungsquelle (28, 29) verbunden sind, welche Messspannungsquellen Messspannungen erzeugen, die zueinander phasenverschoben sind.

6. Probenanalysevorrichtung nach einem der vorhergehenden Ansprüche, **dadurch gekennzeichnet, dass** die Anrege- und die Messelektroden (10, 11, 12, 13) zur kontaktlosen Leitfähigkeitsmessung gegenüber der Innenwand des Separationskanals (1) nach außen versetzt sind.

7. Probenanalysevorrichtung nach einem der vorhergehenden Ansprüche, **dadurch gekennzeichnet, dass** die Anrege- und die Messelektroden (10, 11, 12 13) der zumindest zwei Elektroden-Paare in einer parallel zum Separationskanal (1) verlaufenden Ebene angeordnet sind.

8. Probenanalysevorrichtung nach Anspruch 7, **dadurch gekennzeichnet, dass** die Anrege- und die Messelektroden (10, 11, 12, 13) der zumindest zwei Elektroden-Paare oberhalb des Separationskanals (1) ausgebildet sind.

9. Probenanalysevorrichtung nach einem der vorhergehenden Ansprüche, **dadurch gekennzeichnet, dass** die Anrege- und Messelektroden (10, 11, 12, 13) der zumindest zwei Elektroden-Paare in Längsrichtung des Separationskanals (1) fluchtend angeordnet sind.

10. Probenanalysevorrichtung nach einem der vorhergehenden Ansprüche, **dadurch gekennzeichnet, dass** die Anrege- und Messelektroden (10, 11, 12, 13) der zumindest zwei Elektroden-Paare an einander gegenüberliegenden Seiten des Separationskanals (1) angeordnet sind.

11. Probenanalysevorrichtung nach Anspruch 10, **dadurch gekennzeichnet, dass** benachbart zu den Anrege- und Messelektroden (10, 11, 12, 13) und an einander gegenüberliegenden Seiten des Separationskanals (1) Guard-Elektroden (30, 31, 32, 33, 34, 35, 36, 37) angeordnet sind, welche mit einer Referenzspannungsquelle (94, 95, 96, 97, 98, 99, 100, 101) verbindbar sind.

12. Probenanalysevorrichtung nach Anspruch 11, **dadurch gekennzeichnet, dass** ein Teil der Guard-Elektroden mit den Anregeelektroden (10, 12) und ein weiterer Teil der Guard-Elektroden mit den Messelektroden (10, 11,12, 13) in jeweils einer Ebene angeordnet sind.

13. Probenanalysevorrichtung nach Anspruch 11 oder 12, **dadurch gekennzeichnet, dass** die Guard-Elektroden sowie die Anrege- und Messelektroden (10, 11, 12, 13) entlang der Längsrichtung des Separationskanals (1) angeordnet sind.

14. Probenanalysevorrichtung nach einem der vorhergehenden Ansprüche, **dadurch gekennzeichnet, dass** die Anrege- und Messelektroden (10, 11, 12, 13) planar ausgebildet sind.

15. Probenanalysevorrichtung nach einem der vorhergehenden Ansprüche, **dadurch gekennzeichnet, dass** die Anrege- und Messelektroden (10, 11, 12, 13) und/oder die Guard-Elektroden in der Wand des Separationskanals (1) eingebettet sind.

16. Probenanalysevorrichtung nach einem der vorhergehenden Ansprüche, **dadurch gekennzeichnet, dass** ein Probeneinlass (71, 71') und/oder ein Injektionskanal (73) zum Einbringen der zu analysierenden Probe vorgesehen ist, der mit dem Separationskanal (1) verbunden ist.

17. Probenanalysevorrichtung nach Anspruch 16, **dadurch gekennzeichnet, dass** der Probeneinlass (71) im Anfangsbereich des Separationskanals (1) angeordnet ist.

18. Probenanalysevorrichtung nach Anspruch 16, **dadurch gekennzeichnet, dass** der Probeneinlass (71') ungefähr in der Längsmitte des Separationskanals (1) angeordnet ist, und dass zwischen dem Probeneinlass (71') und den beiden Enden des Separationkanals (1) jeweils zumindest ein Paar von Anrege- und Messelektroden (10, 11, 12, 13) angeordnet ist.

19. Mikrofluidikchip mit einer in einem Substrat (90) ausgebildeten Probenanalysevorrichtung nach einem der Ansprüche 1 bis 18.

20. Verwendung einer Probenanalysevorrichtung nach einem der Ansprüche 1 bis 18 in einem Elektrophoreseverfahren.

21. Verwendung einer Probenanalysevorrichtung nach einem der Ansprüche 1 bis 18 in einem Isotachophoreseverfahren.

22. Verfahren zur Analyse von Proben durch Auftrennung von Probenkomponenten in einem Separationskanal (1), wobei in den Separationskanal (1) zumindest ein Messsignal eingekoppelt wird und zumindest ein Ausgangssignal abgegriffen wird, **dadurch gekennzeichnet, dass** das zumindest eine Messsignal an zumindest zwei in Längsrichtung des Separationskanals (1) voneinander beabstandeten Stellen in den Separationskanal (1) eingekoppelt und zumindest zwei Ausgangssignale abgegriffen werden, die voneinander subtrahiert werden.
